# EUROPEAN PATENT APPLICATION

(11) **EP 1 920 711 A1**
(43) Date of publication of application: **14.05.2008**
(21) Application number: 06255800.2
(22) Date of filing: 13.11.2006
(51) Int. Cl.: A61B 3/06, A23L 1/302

(54) **Assessment and improvement of visual performance**

(71) Applicant: DSM IP Assets B.V., 6411 TE Heerlen (NL)
(72) Inventor: The designation of the inventor has not yet been filed
(74) Representative: Wright, Simon Mark

(57) **Abstract**

A novel method of assessing visual performance is provided, which can simulate the effect of pigment absorption on visual acuity and indicate macular pigment optical density (MPOD). The method can simulate the amounts of pigment in the eye and assess e.g. the response of the eye, (or the reaction of an individual) to different levels of blue light. The individual can be asked to detect a specific feature (a gap in Landolt rings) at decreasing contrast levels, the Landolt rings (and background) both being blue. This can provide an indication of the density or concentration of levels of macular pigments in an individual's eye which can help determine treatments or substances to be administered (carotenoids, such as lutein or zeaxanthin) to improve the individual's visual performance.

## Description

### Introduction

The present application concerns a method of assessing (and, if possible, increasing) visual (which includes contrast) performance (which includes acuity). The method can be used to assess (or provide an indication of) the level or amount of macular pigment in the eye of an individual. The method comprises detecting the response or reaction of the individual, or an individual's eye, to visual stimuli in a certain (or specific) colour, normally in the visible spectrum, for example blue or green.

The invention also relates to a method for increasing visual performance (or acuity) of the eye and treatment and/or prevention of eye disorders, such as age related macular degeneration (AMD). One can assess visual performance or acuity of an individual, and, on the basis of that determination, select or identify (and then administer) a substance to the individual.

### Background of the invention

There are a wide range of disorders or conditions which can result in decreased visual performance. Once particular condition is macular degeneration, particularly age-related, otherwise known as AMD. This conditions impacts millions of older adults every year, and can adversely affect central vision. It can make it difficult for an individual to read, drive or perform other activities. The classic early symptom of AMD is that straight lines appear crooked, and a small blind spot may appear, resulting in the loss of central vision.

Regular eye examinations can detect AMD early, but there is a need to improve the assessment of visual performance and susceptibility to AMD.

The present invention relates to the identification of those subjects which may have poor vision, or low macular pigment (and consequently reduced visual performance) or an increased risk for developing eye disorders, and may benefit from treatment.

### Summary of the invention

The present invention relates to a method of assessing visual (which includes contrast) performance or acuity, or assessing (which includes detecting or determining) the level (or amount) of pigment (such as a macular pigment) in the eye of an individual. This may be done using blue light.

The present invention also relates to a method for improving visual performance (or acuity), or increasing the level (or amount, concentration) of (or e.g. macular) pigment in the eye (which in turn may lead to treatment and/or prevention of an eye condition or disorder, such as age-related macular degeneration, AMD), which comprises:
(a) assessing visual (or contrast) performance (or acuity) of a subject (or individual, the terms are used interchangeably), such as by assessing (e.g. detecting or determining) the level (or amount) of (e.g. macular) pigment in the eye (or assessing performance or response to different levels of blue light); and
(b) providing an effective amount of a (preferably macular) carotenoid, in particular a xanthophyll, such as lutein and/or zeaxanthin and/or vitamin C, vitamin E; beta carotene, zinc and/or copper, and/or or a mixture thereof (the AREDS Cocktail, as described later) to said subject.

The present invention also provides a method of determining a substance to be administered to an individual, the method comprising:
a) assessing visual performance of the individual; and
b) on the basis of the determination in (a), identifying or selecting a substance (or its amount or dosage) capable of improving visual performance in that individual.

The method may additionally comprise:
c) providing (such as administering or communicating) the substance (or its identity, amount or dosage) to the individual.

The invention further provides:
i) a kit for carrying out the method of the invention comprising means for detecting an individual's visual performance in relation to blue light and an effective amount of (a (preferably macular) carotenoid, in particular a xanthophyll, such as lutein and/or zeaxanthin and/or vitamin C, vitamin E; beta carotene, zinc and/or copper, and/or or a mixture thereof (the AREDS Cocktail);
ii) a method of preparing a customised or personalised composition for an individual, the method comprising:
   (a) assessing the visual performance of the individual; and
   (b) preparing a composition suitable for, or tailored to, the individual;
iii) a method of providing a customised composition to an individual that has been assessed for visual performance (e.g. in relation to blue light);
iv) use of a compound in the manufacture of a medicament for improving visual performance in a individual that has been assessed for visual performance in relation to blue light, such as by a method of the invention;
v) a method of improving visual performance, the method comprising administering to the individual an (effective amount of a) therapeutic substance or compound, wherein the individual has been assessed for visual performance, e.g. in relation to blue light, such as by a method of the invention;
vi) a database comprising information relating to visual performance (e.g. in relation to blue light) and optionally their association with (macular) pigmentation and/or substances capable of improving visual performance;
vii) a method for determining whether an individual may be able to improve visual performance, the method comprising:
   (a) inputting data relating to macular pigmentation and/or visual performance to a computer system;
   (b) comparing the data to a computer database, which database comprises information relating to macular pigmentation and/or visual performance and optionally substances therefor; and
   (c) determining on the basis of the comparison whether the individual is susceptible to improvement of visual performance;
viii) a computer program comprising program code means that, when executed on a computer system, instructs the computer system to perform a method according to the invention;
ix) a computer program product comprising a computer-readable storage medium having recorded thereon a computer program according to the invention;
x) a computer program product comprising program code means on a carrier wave, which program code means, when executed on a computer system, instructs the computer system to perform a method according to the invention;
xi) a computer system arranged to perform a method according to the invention comprising:
   (a) means for obtaining or receiving data relating to visual performance of the individual;
   (b) optionally, a module for comparing the data with a database comprising information relating to visual performance and optionally substances for the improvement thereof; and
   (c) optionally, means for determining, on the basis of said comparison whether the individual is susceptible to improvement in visual performance and/or a substance therefor;
xii) a method of preparing a customised composition for an individual, the method comprising:
   (a) assessing visual performance of the individual, such as by a method of the invention;
   (b) determining (such as electronically generating) a customised composition suitable for the individual;
   (c) optionally, generating (e.g. electronic) manufacturing instructions to control the operation of composition manufacturing apparatus in accordance with the customised composition formulation; and
   (d) manufacturing the customised food (according to the manufacturing instructions); or
xii) use of a computer system of the invention to make a customised composition product.

### Description of the Drawings

Figure 1 is a graph of refraction against wavelength and shows the amelioration of chromatic aberration that is ascribed to the macular pigment of the eye, based on its blue light absorbing properties;
Figure 2 is a graph of absorption versus wavelength and shows the overlap between the spectral sensitivity function of rod photoreceptors when compared with the absorption spectrum of macular pigment (the U-shaped line);
Figure 3 shows the targets (Landolt rings) viewed by individuals on a visual display (screen) in order to assess visual performance in accordance with a method of the present invention;
Figures 4A and 4B are graphs of size acuity and contrast acuity thresholds showing a comparison between achromatic and blue stimuli.

### Detailed description of the invention

According to the first aspect of the present invention, there is provided a method of determining a substance to be administered to an individual (or subject, the terms are used interchangeably), the method comprising:
a) assessing the visual performance, e.g. in relation to blue light of the individual;
b) on the basis of the determination in a), identifying a substance capable of improving visual performance, or preventing and/or treating an eye condition or disorder in that individual. The assessment may include determining whether the individual could benefit from (an increased amount of) an eye pigment (e.g. yellow macular pigment).

The method may additionally comprise:
c) providing (e.g. administering or communicating) the substance (or its identity) to the individual (or subject).

Step c) can thus comprise communicating the identity and/or amount (or dosage) of that substance to the individual, for example proposing, suggesting or recommending that substance. For instance, this may involve supplementing a person's food or diet with said substance.

### Assessment of Visual Performance

The method of the invention can comprise measuring (or detecting) the response or reaction of an individual, or an individual's eye, to (visible) light or a visible stimulus (or stimuli). This may be done by performing a series of visual tests that may require response(s), such as to each test, from the individual. The light may be of a certain or specific colour. The light may be provided at two or more different intensities or contrast levels.

The light can be green, but is preferably blue. The visible light (wavelength within 400-700 nm) may therefore have a wavelength of:
(a) from 460-495 nm, such as from 470-480 nm, optimally about 475 nm (blue); or
(b) from 495-535 nm, such as from 505-545 nm, optimally about 510 nm (green).

Preferably, the light is of a wavelength such that it may be absorbed by one or more pigments in the eye. The pigment may be a macular pigment, for example lutein, zeaxanthin and/or meso-zeaxanthin.

The method may comprise determining a person's reaction or response to a visual display, such as by one or more visual tests. This display may be a screen, for example attached to or driven by a computer. The display may have one or more (first) shape(s) presented in a desired colour or within the desired wavelength. The display may thus show one or more rings or circles, for example Landolt rings. Such shapes (e.g. rings) may be green, but are preferably blue. Suitably, there are at least two or more (a plurality of) rings, such as three, four or five rings, preferably four. One of those rings may contain a gap, in other words it may not be closed, or incomplete. The individual may be assessed to see whether they can determine a feature in one of the shapes, for example which (usually one) ring contains the gap and/or where in the ring a gap is located. This (test) may be repeated two or more times, for example at different (e.g. decreasing) intensities or contrast levels. Preferably, the individual is tested firstly to see which ring contains the gap, and (e.g. secondly and if necessary) where in the ring the gap is located.

The display may also show a (different, e.g. second) shape in (e.g. another) colour (e.g. red) such as a (filled) circle or spot or dot. This may be near the middle of the display, or between at least two rings. Preferably the other shape (e.g. spot) is coloured red and/or has a wavelength within 700-620 nm, e.g. about 640-660 nm, preferably about 650 nm. This (e.g. red dot) can be placed centrally and can provide a focus for the eye. It can thus act as a reference or fixation point for the individual. The individual may be asked to look at, fix or focus on this (second) shape (red dot) so that peripheral vision can be assessed (for example by use of Landolt rings). The (second) shape can act as a fixation spot.

The method may be used to simulate or assess the amount of pigment in the eye. Thus the invention broadly relates to assessing the performance or acuity (visually) to different levels of (e.g. blue) light or can be predictive of MPOD.

The light may be varied in intensity, contrast and/or level. This may be done continuously, but is preferably in steps, or stepwise, so a plurality of visual tests are set or performed. The individual may therefore be provided with different intensity or levels of the visible light (usually of the same colour or similar wavelength). The individual's response to the different levels of light (e.g. tests) can be determined. This may give a measurement of the visual or contrast acuity. The assessment can be used to provide an indication of macular pigment levels in the eye.

The assessment may therefore comprise a plurality or series of (usually related) visual tests. Preferably, the visual display will have a background that is blue (or within a wavelength of 460-495 nm). The (e.g. first) shape, for example one or more rings, may also be of the same colour, or wavelength, or at least within 10, preferably 5 nm of the background. Thus, in preferred embodiments, both the background of the visual display and the first shape (e.g. Landolt rings) are both blue.

Initially, or in a first test, the (first) shape will have a different intensity or contrast level to the background, to enable it to be seen, or to be visible to the individual who is being assessed. Over time, the different intensity or contrast level can be reduced. The individual's response to decreasing contrast levels or intensities (for example as compared between the shape and background) can be decreased or diminished over time, and in subsequent tests. The individual's ability or response on the (subsequent) tests can be measured. Indeed, the entire assessment can be performed by a computer.

Thus, the individual may be provided with a series of tests where the intensity or contrast of the (e.g. first) shape, in comparison or with respect to the background, is diminished. This will make the shape harder to see, over time, and in subsequent tests. The individual's ability to detect or see a particular feature in the shape can therefore be measured. At some point the individual is likely to stop being able to see the feature in the shape, in other words, the shape becomes almost indistinguishable from the background. This can provide a threshold or cut-off level for measurement. This can be used to assess the individual's visual performance, and provide measure of acuity. Clearly the more tests that the individual can pass, in other words the ability to detect a feature in the (first) shape at lower contrast levels can provide a measure of low contrast level and will indicate increased acuity.

The individual's reaction or response, for example to each test, can be measured by requiring the individual to detect the feature in the shape (for example, the gap in the Landolt ring). The individual may be required to indicate which (usually one) ring (or shape) contains the gap (or distinguishing feature).

As described before, there may be one or more (first) shapes, only one of which has a (distinguishing) feature (such as a gap) which the individual is asked to detect. There may be thus a plurality of these first shapes, preferably four. These may be provided in quadrants on the display or screen. Thus, the four shapes may be in the top and bottom, and left and right, of the display. Only one may contain the feature (such as a gap) which the individual will be required to detect. The feature may vary between tests, for example it may appear in different shapes on the visual display in different (e.g. consecutive) tests. Thus, in a preferred embodiment, the feature (gap) may appear in different Landolt rings in each test.

The individual can indicate which of the shapes (e.g. four rings) has the distinguishing feature either orally or usually by touch, for example using a computer keyboard or appropriate response device, for example a response button box. There may be one or more buttons corresponding to the number of the shapes (such as four buttons for four shapes). The buttons may correspond to the position of the shapes (for example four, in different quadrants, top left, top right, bottom left, bottom right).

The series of tests may take approximately two minutes for completion. Preferably a computer will have noted and monitored all the individual's responses to the component tests. A next or subsequent test may only be provided to the individual once he has responded to the previous test.

One can provide low photopic level of light (rod dominated) and/or (high) mesopic light (cones and rods) and preferably both, e.g. in a series of tests. The individual's response to the two (or more) intensities or contrast levels (e.g. of blue) light can lead to the assessment and, if necessary, an indication of macular pigment optical density (MPOD).

The invention also relates to a (visual) display for use in performing the assessment method of the invention. The display may show, or be capable of showing, one or more shapes as described above. These shapes are preferably rings or circles. At least one of the rings may be incomplete, or have a gap in it. Preferably there are four rings, or the display may show Landolt rings. Suitably one of these shapes (such as the ring) is blue, or is of a colour having a wavelength, suitably in the visible spectrum, of from 460-495 nm.

### Visual Display

The present invention therefore also relates to a visual display, such as a (e.g. computer) screen or VDU, showing a plurality of shapes. Such shapes may be rings, such as Landolt rings, although they may be alternative shapes, such as a circle with a segment removed. Only one of the shapes may have a distinctive or distinguishing feature which the individual may be asked to detect. The display may therefore have:
(a) a plurality of shapes where at least one of the shapes has a distinctive feature for detection, for example the shapes comprise rings and only one of the rings is an incomplete circle, or has a gap, at least one of the shapes (preferably all of them) have a colour within the wavelength range of 460-495 nm (such as being coloured blue);
(b) the background (to the shape or shapes) has a wavelength of from 460-495 nm, for example the background is blue; and/or
(c) there are three, preferably four, shapes, such as rings, and only one of these shapes has a distinctive and/or detectable feature which the individual will be asked to detect.

Suitably four shapes will be displayed (such as four rings). They may be placed in quadrants, as described above (top left, top right, bottom left, bottom right). Preferably the visual display will also show a (second) shape, such as a (e.g. full) circle or spot or dot, preferably coloured red. This second shape may be located centrally, or at least between the first shapes. Preferably it is located in the middle of four shapes (such as Landolt rings). This may constitute a fixation spot.

The display may be driven by or connected to a computer. The display may be able to vary the intensity or contrast of the shapes (e.g. rings) which can be blue. It may therefore present the individual with a series of images, over time, the images (e.g. rings) for example differing ring in the intensity or contrast of e.g. blue light. The individual may be provided with images of decreasing intensity of the light. One can assess whether the individual can detect, for example, a gap in the ring and/or where in the ring in the gap is located. The cut-off point, or threshold, when the individual can no longer (or with difficulty) detect the gap, can be used as an indication of pigment density and visual performance. Therefore, the assessment method can effectively result in a data acquisition process in order to detect an individual's stability to notice or detect a feature of an image that is, for example, coloured blue.

The assessment may additionally comprise obtaining relevant information from, or about, that individual. That information may be personal and/or clinical information. The information may relate, directly or indirectly, to visual performance or eye disorder(s). Such information may comprise information concerning lifestyle, health, nutritional status, diet. Other personal information of relevance can include age, sex, weight and/or ethnic background. Clinical information may comprise current drug and/or vitamin regimes, current or past treatments, familial data, health risks, family background, medical conditions and/or allergies. It may therefore involve obtaining a patient's history, and determining their nutritional profile. The individual may be able to provide this information, for example, by completing a questionnaire, for example one provided by a computer.

### Optical Assessment

The assessment is thus usually accomplished optically, and can involve measuring light absorption, e.g. by a macular pigment or other suitable optical measurement in or of the eye (e.g. the retina). This can provide an indication of the level of a carotenoid, usually a macular carotenoid (such as lutein or zeaxanthin) in the eye of the individual.

In a preferred embodiment of the invention, the (profile of) macular pigment optical density (MPOD) can be predicted or measured. This can involve determining spatial profiles of the density of the yellow macular pigment across the retina. On a visual display, the subject can view a target(s). One component, the blue light, is absorbed by the macular pigment, whilst another component, which appears orange to the eye, is not absorbed by the macular pigment.

The assessment is in essence a data gathering step to provide an indication of visual acuity. This may be affected by various factors such as age, eye condition (medical disorders or otherwise), ambient light levels, etc. It does not immediately lead to one particular (or, indeed, any) specific method of treatment or course of action.

### Communication of substance

The nature or identity of the substance can be communicated either (e.g. directly) to the individual, or their doctor, optician, physician, guardian, dietician or (genetic) counselor. The communication may be electronically, for example via a computer (a PC or a laptop), portable computer or mobile phone or using the internet. Alternatively, it may be communicated on paper, for example in a booklet or information pack.

The communication of the nature or identity of the substance may be provided through a handheld, pocket or bracelet, watch-type device, personal computer, a personal digital assistance (PDA), a device which may be attached to or integral with a shopping cart or trolley, a terminal to an on-line service (e.g. in an outlet or retail store, such as a super/hypermarket), for example through the internet, a telephone with voice communication, kiosk or centralised computer system. The assessment of visual performance may (also) be achieved by a computer (which may be the same or different from that as just described).

### Substances and compositions to be provided to the individual

The substance or composition may comprise a compound, such as an active ingredient, a drug, pharmaceutical or nutraceutical. The substance may be edible and/or comprise a food, foodstuff or feed, for example a (dietary) supplement, or pharmaceutical composition.

The substance (or composition) may be in any form, for example suitable for oral administration, such as in solid form such as tablets, including effervescent tablets, soft or hard-shell capsules, or in liquid form such as solutions or suspensions, such as an oily suspension. Besides any active ingredient, the preparation may contain one or more conventional (e.g. pharmaceutical) carrier materials, additives and adjuvants, for example, including one or more of gelatine, vegetable gum, sugar, vegetable oil, polyalkylene glycol, flavouring agent, preservative, stabilizer, a emulsifying agent and/or a buffer. The substance, if medicament, can be a controlled (or delayed) release formulation.

The (therapeutic) substance may be administered in various manners such as orally, intracranially, intravenously, intramuscularly, intraperitoneally, intranasally, intrademally, and subcutaneously. The pharmaceutical compositions that contain the therapeutic agent will normally be formulated with an appropriate pharmaceutically acceptable carrier or diluent depending upon the particular mode of administration being used. For instance, parenteral formulations are usually injectable fluids that use pharmaceutically and physiologically acceptable fluids such as physiological saline, balanced salt solutions, or the like as a vehicle. Oral formulations, on the other hand, may be solids, for example tablets or capsules, or liquid solutions or suspensions. In a preferred embodiment, the therapeutic agent is administered to the individual in their diet, for example in a drink or food.

The present invention may thus provide an optimisation of diet and or nutritional supplementation and or pharmaceutical administration, based on the assessment of visual performance. The optimisation, for example of nutrition or nutritional supplementation, may be for a group of individuals, usually related ones, such as a family.

If the substance is a nutritional supplement, this may include foods, capsules, pills, powders, gums and liquids or other oral dosage forms. Also encompassed are nutritional supplements that can be delivered for example to the digestive system, or intravenously, as well as supplements that can be administered through other routes, such as mucous membranes. The individual supplements may comprise excipients, impurities or other components other than the substance of interest.

Once the individual's visual performance has been assessed, one can optimise the nutritional intake, in particular of the substance or composition. In this sense the identity of the substance itself, the amount, dosage and the form in which it is ingested or administered can be tailored to that individual, so that the substance is personalised for that particular individual. The result of the assessment may be to include a proposal to reduce intake of supplement, macronutrient or foodstuff, as well as increasing or adding a substance or other nutritional substance.

Once the individual has been assessed, an effective amount of a (preferably macular) carotenoid, such as lutein, zeaxanthin and/or meso-zeaxanthin, and/or the AREDS cocktail (a component thereof) can be suggested or administered. The substance can be a xanthophyll (for example, a carotenoid possessing one or more oxygen atoms, such as an - OH or hydroxy group).

An effective amount of the carotenoid can be used. Preferably this is lutein and/or zeaxanthin and/or "the AREDS cocktail" (vitamin C, vitamin E, beta carotene, zinc and/or copper, AREDS Report No. 8, Arch. Ophthalmol. 2001;119:1417-1436, referred to as "AREDS Cocktail", also at HKJ Ophthalmol. Vol. 4, Nr. 1, (2000), p. 31-42) and/or one of the components of the AREDS cocktail. For the purposes of the present invention this can be e.g., within the range of from 0.001 mg per kg body weight to about 20 mg per kg body weight. More preferred is a daily dosage of about 0.01 to about 10 mg per kg body weight, and especially preferred is about 0.1 to 1.0 mg per kg body weight per day, especially for the carotenoid, e.g. lutein and/or zeaxanthin. Preferably the carotenoid, e.g. lutein and/or zeaxant lutein and/or zeaxanthin hin are administered at a dosage of from 1 or 5 to 15, 30 or 50 mg/day, such as from 8 or 10 to 12, 15 or 20mg/day and may be present in compositions at that (daily) dosage. Preferred compositions can contain from 8 to 12mg lutein or zeaxanthin (and preferably both within this range).

Especially preferred for vitamin C is 1 to about 10 mg per kg body weight, for beta-carotene 0.1 to about 0.3 mg per kg body weight, for vitamin E 1IU to about 10 IU per kg body weight, for zinc 0.1 mg per kg body weight to about 1.5 mg kg body weight, and for copper 0.01 mg per kg body weight to about 0.05 mg per kg body weight. Zinc is preferably used as zinc oxide and copper as cupric oxide.

Preferable daily dosages and/or amount in an oral (e.g. daily) formulation, such as a tablet, are as follows. The formulation may comprise an antioxidant. This may be vitamin C (such as at from 200 to 800mg, 400 to 600mg, such as 450 to 550mg). There may be 1, 2 or 3 antioxidants present. In addition or alternatively, another antioxidant is vitamin E. This may be present at a dosage of from 100 to 700 IU, such as from 200 to 600 IU, preferably from 300 to 500 IU. Another preferred antioxidant, instead of or in addition to vitamins C and E, is beta-carotene. Beta-carotene may be present at from 5 to 40mg, such as from 10 or 20 to 30 or 40mg, preferably from 13 to 18 mg. The zinc may be present as zinc oxide, and can be an amount of from 20 to 140mg, such as from 60 to 100mg, preferably from 70 to 90mg. The copper may be present at from 1 to 2mg.

The duration of the treatment can be suitably life-long, and no shorter than the above-mentioned markers would indicate or suggest that the subject involved is no longer at risk for developing AMD or still suffers from AMD. Suitably, treatment is started with an initial dosage of 0.5 - 1.0mg of carotenoid (e.g. xanthophyll), such as lutein and/or zeaxanthin, per kg body weight per day for 1-2 months whereupon the dosage may be lower to secure a macular pigment optical density of three times the threshold value, i.e. 0.6.

Preferably, two or more xanthophylls are present, such as a combination of lutein and zeaxanthin. In such combination these compounds are preferably used in a ratio of 0.1-1.0 : 1.0- 0.1 parts (by weight), such as from 0.5-1.0:1.0-0.5, especially 0.9-1.1:0.9-1.1, to each other.

In accordance with the invention, the substance, such as lutein and/or zeaxanthin and/or the "AREDS Cocktail" or its individual components can be provided in any appropriate form, suitably for oral administration, e.g. as a pharmaceutical composition, or in food or beverage. The term "providing" as used herein is to be understood as denoting the act of collecting the desired active ingredients and processing them into a suitable administration form, as well as the direction for use and/or administration to the subject involved. Higher dosages and amounts can be provided to individuals who appear to be at greater risk, for example one of more polymorphisms associated or related to AMD, and so one can correlate higher dosages with greater risk (or more polymorphisms)

In still another aspect, the invention relates to the use of a (preferably macular) carotenoid, e.g. xanthophyll, such as lutein and/or zeaxanthin and/or a vitamin C, beta-carotene, vitamin E, zinc and copper or a mixture thereof in the manufacture of a medicament for the treatment and/or prevention of age-related macular degeneration (AMD) in a subject which has been identified as being at risk of developing AMD, or as suffering from AMD, especially by one of the methods (of the invention) identified above.

### Databases and foods/compositions

In the assessment of an individual, one can obtain personal data, which may be obtained through automated data analysis, interview survey subjective analysis and/or laboratory testing. A database can be provided with information concerning available nutritional supplements, including contents, price and dosage form. A further database may include information, including risks and benefits, about constituency of nutritional supplements, for example information concerning the substance.

The invention can further include apparatus for formulating the substance, for example in a food or in a nutritional supplement, usually based on the determination of susceptibility. A specific formulation may then be provided or communicated to the individual, which may (or may not) be standard dosage form. The invention thus additionally contemplates a vending machine or point of sale dispensing machine (such as comprising a computer) which can formulate, or combine, pre-prepared dosage forms of nutritional supplements, based on the proposed nutritional supplementation or the substance to be taken by the individual.

The (e.g. the point of sale dispensing) machine is in a public location, an interface may be provided, for example a (touch) screen. This screen may also be used to perform the visual assessment as described previously. Optionally, an individual may be interviewed, optionally in the presence of a trained professional, with the data inputted or accepted in an appropriate format. Thus, a trained professional, such as optician, doctor, nurse, chiropractor, social worker or nutritionist may assist in the input in medical information, etc.

### Customised composition (e.g. food)

In one aspect, the invention relates to a customised diet for an individual that is susceptible to improvement in their visual performance.

Accordingly, the present invention enables the preparation of a customised composition (or diet) suitable for such an individual, wherein the customised composition or diet comprises one or more ingredient(s) that can improve visual performance and/or does not comprise components that are adverse to visual performance. The preparation of customised food may be carried out using electronic means, for example by using a computer system.

In one embodiment, the composition may be formulated to alter the profile of food proteins in order to minimise the potential for secondary dietary sensitivity. The customised food may be hypoallergenic and/or may exclude ingredients that are poorly tolerated or cause allergies, for example gluten-containing grains such as wheat, particular protein sources such as animal proteins, milk (lactose), eggs, soy, peanuts, shellfish, fruits or tree nuts.

In another embodiment, the (customised) composition may be formulated to include functional or active ingredients that help improve visual performance.

The present invention also relates to a method of providing a composition (e.g. food) suitable for an individual who has been determined to improvement in visual performance such as by an assessment method of the invention.

The customised composition can be made to an inventory and supplied from inventory, i.e. is pre-manufactured rather than being made to order. Therefore the composition may not be specifically designed for one particular individual but may be suitable for a relative of the individual that may also be susceptible to age-related macular degeneration (or an age-related macular degeneration-related disorder). Alternatively, the composition may be suitable for a group of individuals that are susceptible to an age-related macular degeneration-related disorder, such members of a family. In preferred embodiment, the composition is personalised or customised to meet the nutritional requirements of a specific individual.

### Bioinformatics

The data or information concerning visual performance may be stored in an electronic format, for example in a computer database. Accordingly, the invention provides a database comprising information relating to visual performance, which may include further information about the eye disorders or conditions. The database can comprise information regarding the substance(s), which are suitable and/or not suitable, for individuals (e.g. whose visual performance can be improved).

A database may be used to assess the level of visual performance and whether it can be improved (and, for example, in which respect). Such an assessment may be carried out by electronic means, for example by using a computer system (such as a PC). Typically, the assessment will be carried out by obtaining or inputting data from or relating to the individual concerning visual performance (such as the results of the assessment, e.g. in relation to blue light) to a computer system; comparing the genetic data to a database comprising information relating to visual performance; and, if necessary, on the basis of this comparison, determining the susceptibility of the individual to improvement in visual performance.

The invention also provides a computer program comprising program code means for performing all the steps of a method of the invention when said program is run on a computer. Also provided is a computer program product comprising program code means stored on a computer readable medium for performing a method of the invention when said program is run on a computer. A computer program product comprising program code means on a carrier wave that, when executed on a computer system, instruct the computer system to perform a method of the invention is additionally provided.

The invention also provides an apparatus arranged to perform (e.g. the assessment) method according to the invention. The apparatus typically comprises a computer system, such as a PC. In one embodiment, the computer system comprises: means for performing the assessment on the individual; optionally a module for comparing the data with a database comprising information relating to visual performance; and, if necessary, means for determining on the basis of said comparison the susceptibility of the individual to improved visual performance.

### Composition/food manufacturing

In one embodiment of the invention, the manufacture of a customised composition may be controlled electronically. Typically, information relating to the assessment of an individual may be processed electronically to generate a customised composition. The customised composition may then be used to generate electronic manufacturing instructions to control the operation of composition manufacturing apparatus.

The apparatus used to carry out these steps will typically comprise a computer system, such as a PC, which comprises means for processing the nutritional information to generate a customised composition; means for generating electronic manufacturing instructions to control the operation of composition manufacturing apparatus; and a composition manufacturing apparatus.

The composition manufacturing apparatus may comprise a packaging apparatus. The packaging apparatus typically packages the composition into a container (such as a plastic or paper bag or box). The apparatus may also comprise means for labeling the composition, typically after packaging. The label may provide information such as: ingredient list; nutritional information; date of manufacture; best before date; weight; and types of individual(s) for which the composition is suitable.

Preferred features and/or characteristics of one aspect of the invention are applicable to another aspect mutatis mutandis.

The invention is illustrated further by the Examples given below:
course of lutein and zeaxanthin, both at dosages of 10mg/day.

### Example 1

The invention relates to a novel technique to simulate increased MPOD (Macular Pigment Optical Density) in order to evaluate its effect on visual performance

This new technique combines two effects into one experiment that can reveal potential improvements in visual performance upon supplementation. Both effects relate to the selective absorption of blue light by the macular pigment and are based on the following two factors:
1. Reducing the "blue" light content of broadband stimuli improves visual acuity by reducing chromatic aberration; and
2. Selective reduction of rod photoreceptor signals in the mesopic range over a small region of the retina that is dominated by cone photoreceptors helps to extend the superior characteristics of cone vision into the twilight zone.

### Chromatic aberration

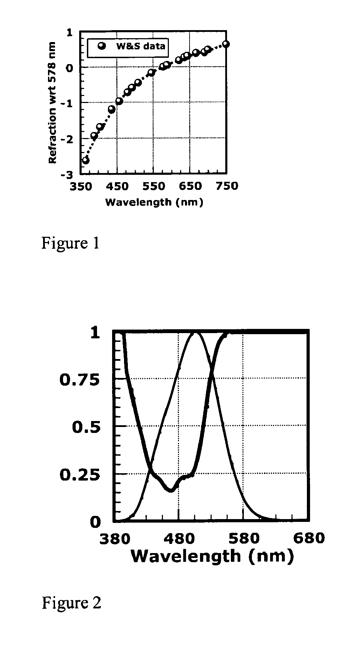

One of the more common aberrations optical systems show is chromatic aberration. It occurs because lenses refract light of shorter wavelengths stronger than light of longer wavelengths, with the result that, if the red colour of an image is in perfect focus on the retina, the blue part of the picture is in focus only in front on the retina, with other words: the blue part of the image on the retina is blurred. The amelioration of chromatic aberration is one of the "classical" functional roles ascribed to the macular pigment, based on its blue light absorbing properties (see Figure 1).

### Reduction of rod photoreceptor signals

Macular pigment may help to maintain the advantages of cone mediated vision at lower light levels. The light sensitivity of cones is higher than that of rods. On the other hand, cones and not rods are responsible for high resolution vision because they are much denser packed than rods. As ambient light decreases, cones become more and more insensitive until in the twilight zone (the mesopic range) rods are starting to be activated. However, the low resolution rods deteriorate the sharp cone picture and, therefore, it would, theoretically, be helpful to prevent rods to spoil the sharp cone picture. The absorption spectrum of the macular pigment overlaps to a large extent with the sensitivity function of rods (see Figure 2), so that macular pigment could indeed "switch off' cones in the mesopic range. This is a key factor.

Figure 2 shows the overlap between the spectral sensitivity function of rod photoreceptors (bottom line, with peak) and the absorption spectrum of macular pigment (U-shape line, with trough).

### Basic Concept

The appropriate use of a calibrated visual display that incorporates a "notch" filter (to narrow down the spectral composition of the blue and green display phosphors) and a spectrally calibrated neutral density filter (to secure stable display operation at low light levels) makes it possible to simulate the effect of macular pigment absorption on visual acuity.

We simulated a number of different macular pigment density levels and measure visual acuity and/or contrast acuity for each level of macular pigment at the fovea (where the effects of chromatic aberrations are likely to affect acuity most) and at an eccentricity of 0.75° (still close to the center of the fovea where acuity remains high but where rod photoreceptor signals begin to affect visual performance at lower light levels).

The experiments were repeated using a number of light levels to cover both low photopic (i.e., cone dominated vision) and high mesopic (i.e., when vision relies on both cone and rod photoreceptor signals). The experiments were carried out with a number of volunteers. It was demonstrated that the effects mentioned above can affect the most important aspect of visual performance (i.e., visual acuity) and to provide indication of the level of macular pigment absorption of blue light needed to achieve a significant effect.

### Development and optimization of the new test and measurement procedure

The new test is a modified CAA Test (Contrast Acuity Assessment) where subjects viewed a set of four blue Landolt rings on a computer display while fixating the red spot in the middle. Only one of those four rings has a gap. The individual determines firstly where the Landolt ring with a gap is located (top right or left, or bottom right or left), and, secondly where within this ring the gap is located (top right or left, or bottom right or left).

The test was performed with different levels of blue light in the Landolt rings to simulate different amounts of macular pigment. For each simulated macular pigment level, visual performance will be estimated by the correct allocations the subjects have made during a test session.

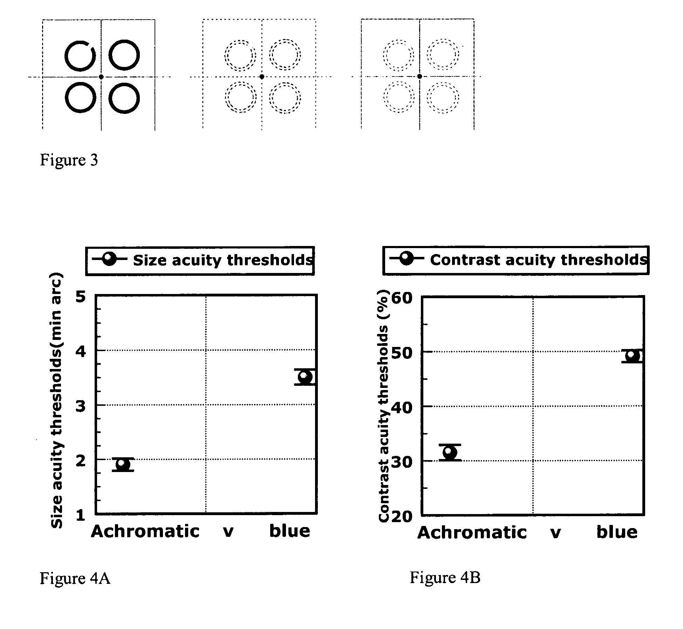

Figure 3 shows the targets viewed by the individuals. From left to right a increasing MPOD was simulated, with the effect that the rings at the far right appear almost colourless.

### Example 2 contrast acuity assessment with stimuli rich in blue light)

In a related study visual acuity was assessed by determining the size threshold of visibility of the gap in a Landolt ring in a similar manner as described in Example 1. The thresholds were measured in minutes of arc (min arc).

The preliminary measurements showed the differences between achromatic and blue rich stimuli on size and contrast acuity thresholds.

The test parameters were as follows:

### Background

Select uniform background (CIE A) at a moderate luminance (~ 16 cd/m²) so as to generate largish pupil size and to encourage accommodation on fixation and guides defined in middle & long wavelength light.

### Fixation stimulus

The fixation spot and the guides had high luminance contrast and were rich in long wavelength light (same composition as CIE illuminant A) so as to enhance chromatic difference of focus.

### Test stimulus

The test stimulus contained a lot of blue light (modulation orientation ~ 233.3° orientation, towards blue phosphor location (0.1488, 0.0758) on the CIE - (x,y) chart), but the gap position was detected by means of luminance contrast (largely through activation of L and M cones). One can measure contrast detection thresholds (fixed target size of 10 min arc), or acuity thresholds (when we vary target size) using fixed luminance contrast of 24%.

### Method

Landolt ring stimulus, 4 alternative, forced-response procedure ~ 9 reversals, time ~ 2 minutes.

The results demonstrate a reduction in visual acuity for blue targets, evidenced by substantial higher thresholds for blue rich stimuli (see right side of Figure 4A or 4B) than for achromatic stimuli (see left side of Figure 4A or 4B) when viewed against a middle to long wavelength rich background. The stimulus conditions were selected to demonstrate the effects of chromatic aberration in the eye and the contrast of the Landolt ring target was set at the same value for both the achromatic and the blue targets.

### Examples 3 and 4

An individual was assessed for visual performance in relation to blue light as described in Example 1. On the basis of this assessment an indication of MPOD (macular pigment optical density)was created. With information concerning macular pigment density, soft gelatin capsules to be administered according to an individual were prepared comprising the following ingredients:

| Ingredient | Amount per Capsule |
|---|---|
| Lutein | 8 and 10 mg |
| Lecithin | 50 mg |
| Soy bean oil | 200 mg |

One or more capsules may be taken, suitably with breakfast.

### Examples 5 and 6

An individual was assessed for visual performance in relation to blue light as described in Example 1. On the basis of this assessment an indication of MPOD was created. With information concerning macular pigment density, soft gelatin capsules were prepared comprising the following ingredients:

| Ingredient | Amount per Capsule |
|---|---|
| Lutein | 8 and 10 mg |
| Zeaxanthin (or meso-zeaxanthin) | 8 and 10 mg |
| Lecithin | 50 mg |
| Soy bean oil | 200 mg |

### Example 7

Soft gelatin capsules were prepared for an individual who had undergone the visual assessment according to Example 2 comprising the following ingredients:

| Ingredient | Amount per Capsule |
|---|---|
| Lutein | 6 mg |
| Zeaxanthin | 6 mg |
| Lecithin | 50 mg |
| Soy bean oil | 200 mg |

### Example 8

Soft gelatin capsules were prepared for an individual who had undergone the visual assessment according to Example 2 comprising the following ingredients:

| Ingredient | Amount per Capsule |
|---|---|
| Lutein | 12 mg |
| Lecithin | 50 mg |
| Soy bean oil | 200 mg |

### Example 9

Soft gelatin capsules was prepared for an individual who had undergone the visual assessment according to Example 2 comprising the following ingredients:

| Ingredient | Amount per Capsule |
|---|---|
| Zeaxanthin | 12 mg |
| Lecithin | 50 mg |
| Soy bean oil | 200 mg |

### Example 10

Soft gelatin capsules were prepared for an individual who had undergone the visual assessment according to Example 2 comprising the following ingredients:

| Ingredient | Amount per Capsule |
|---|---|
| Lutein | 12 mg |
| Zeaxanthin | 12 mg |
| Lecithin | 50 mg |
| Soy bean oil | 200 mg |

### Examples 11 to 17

Eight soft gelatin capsules were prepared in advance for individuals with differing visual performance levels as assessed by the procedure of Example 2, comprising the following ingredients:

| Ingredient | Amount per capsule | | | | | | |
|---|---|---|---|---|---|---|---|
| Lutein | 6mg | 6mg | 8mg | 8mg | 10mg | 12mg | 12mg |
| Zeaxanthin | 6mg | 6mg | 8mg | 8mg | 10mg | 12mg | 12mg |
| Vitamin C | 500mg | 500mg | 600mg | 700mg | 400mg | 300mg | 400mg |
| Beta-carotene | 20mg | 300mg | 15mg | 15mg | 15mg | 10mg | 10mg |
| Vitamin E | 400IU | 450IU | 500IU | 500IU | 500IU | 300IU | 300IU |
| Zinc (as zinc oxide) | 80mg | 60mg | 70mg | 70mg | 60mg | 50mg | 40mg |
| Copper (as cupric acid) | 2mg | 3mg | 4mg | 4mg | 3mg | 2mg | 2mg |
| Lecithin | 50mg | 60mg | 70mg | 50mg | 60mg | 70mg | 50mg |
| Soy bean oil | 200mg | 220mg | 250mg | 250mg | 200mg | 150mg | 50mg |

## Claims

1. A method of assessing visual (or contrast) performance (or acuity), or assessing the level of pigment in the eye of an individual, the method comprising detecting the reaction of the individual, or of the individual's eye, to visible light.

2. A method according to claim 1 when the light is of a specific colour and/or is provided at two or more intensities or contrast levels.

3. A method according to claim 1 or 2, wherein the visible light is green, or preferably blue and/or has a wavelength within 495-535 nm or within 460-495 nm.

4. A method according to any preceding claim, wherein the pigment is a macular pigment.

5. A method according to any preceding claim, wherein the visible light can be absorbed by a pigment in the eye.

6. A method according to claim 4 or 5, wherein the pigment comprises lutein and/or zeaxanthin.

7. A method according to any preceding claim comprising detecting a reaction or response (of the individual) to a visual display.

8. A method according to any preceding claim, wherein two different intensities or contrast levels of the light is provided, and the reaction or response to two or more (or each) intensity or contrast level is assessed.

9. A method according to claim 8, wherein the reaction or response of the individual, or the individual's eye, is detected to two or more intensities or levels (such as low photopic and/or high mesopic) of the light.

10. A method according to any preceding claim which additionally comprises assessing the density or concentration of a pigment in the individual's eye.

11. A method according to any preceding claim which comprises providing a (visual) display (to the individual) on a screen, such as connected to or driven by a computer.

12. A method according to any preceding claim, wherein the individual is shown a display with one or more rings, such as Landolt rings.

13. A method according to claim 12, wherein the Landolt rings are blue in colour and/or have a wavelength of from 470-800 nm.

14. A method according to claim 12 where there are four Landolt rings, at least one of which is not closed or contains a gap.

15. A method according to claim 12, wherein the display additionally comprises another shape (e.g. a spot), preferably near the middle of the display or between 2 or more rings, optionally of a different colour to (one or more of) the rings.

16. A method according to claim 15, wherein the shape (e.g. spot) is red and/or has a wavelength of from 640-660 nm.

17. A method according to claim 12, wherein the individual is assessed to see whether they can determine which ring contains the gap and/or where in the ring the gap is located.

18. A method according to any preceding claim which comprises simulating the amount of pigment in the eye, simulating the effect of pigment absorption on visual acuity and/or assessing visual performance at different levels or intensities of blue light.

19. A method for improving visual performance which comprises:
(a) assessing the (individual) visual performance of a subject; and
(b) providing an effective amount of a carotenoid and/or vitamin C, vitamin E; beta carotene, zinc and/or copper, and/or or a mixture thereof (the AREDS Cocktail) to said subject.

20. A method according to claim 19 wherein the carotenoid comprises a xanthophyll.

21. A method according to claim 19 or 20 wherein in step (b) 0.001 mg to 20 mg, preferably 0.1 mg to 1.0 mg of carotenoid (lutein, zeaxanthin and/or meso-zeaxanthin) is administered per kg body weight per day.

22. A method of any of claims 19 to 21 wherein in step (b) 0.001 mg to 20 mg, preferably 0.1 mg to 1.0 mg per kg body weight of lutein and/or zeaxanthin plus vitamin C (1 to about 10 mg per kg body weight), beta-carotene (0.1 to about 0.3 mg per kg body weight), vitamin E (1IU to about 10 IU per kg body weight), zinc (0.1 mg per kg body weight to about 1.5 mg kg body weight), copper (0.01 mg per kg body weight to about 0.05 mg per kg body weight) is administered per day.

23. The use of a carotenoid, such as lutein and/or zeaxanthin, and/or a mixture of vitamin C, beta-carotene, vitamin E, zinc and copper in the manufacture of a medicament for the improvement of visual performance in a subject who has been assessed for visual performance, such as in relation to blue light.

24. The use of claim 23 wherein the medicament contains an amount of carotenoid, such as lutein/and or zeaxanthin, which is sufficient to administer 0.001 mg to 20 mg, preferably 0.1 mg to 1.0 mg of lutein and/or zeaxanthin per kg body weight per day.

25. A method of determining a substance to be administered to an individual, a method comprising:
a) assessing the visual performance of the individual, such as in relation to blue light, optionally with an assessment method of any of claims 1 to 18; and
b) on the basis of the assessment in (a), identifying a substance capable of improving visual performance in that individual.

26. A method according to claim 24 additionally comprising:
c) providing (such as administering or communicating) the substance (or its identity) to the individual.

27. A method according to claim 24 or 25 wherein in (b) a proposal, suggestion or recommendation is made to the individual concerning the substance to be administered.

28. A method according to any of claims 25 to 27 wherein the substance is a compound (such as drug, pharmaceutical, or nutraceutical), a foodstuff, feed or dietary supplement.

29. A method of preparing a customised composition for an individual, the method comprising:
(a) assessing the visual performance of the individual; and
(b) preparing a composition suitable for, or tailored to, the individual.

30. A method of providing a customised composition, the method comprising providing a composition suitable for the individual to improve visual performance, wherein the individual has been assessed for visual performance, such as in relation to blue light.

31. Use of a compound which is an optical therapeutic in the manufacture of a medicament for improving visual performance in an individual that has been assessed for visual performance by a method according to any one of claims 1 to 18.

32. A computer system arranged to perform an assessment method according to any of claims 1 to 18 comprising:
(a) means for obtaining or receiving data relating to visual performance of the individual;
(b) optionally, a module for comparing the data with a database comprising information relating to visual performance; and
(c) optionally, means for determining on the basis of said comparison whether the individual can benefit from increased visual performance or means for identifying or providing a substance to the individual.

33. A method of preparing a customised composition for an individual, the method comprising:
a) assessing the individual's visual performance by a method according to any of claims 1 to 18;
(b) (e.g. electronically) generating a customised composition suitable for the individual;
(c) optionally, generating electronic manufacturing instructions to control the operation of composition manufacturing apparatus in accordance with the customised composition; and
(d) manufacturing the customised composition (for example, according to the electronic manufacturing instructions).

34. A computer system according to claim 32, further comprising:
(e) means for electronically generating a customised composition formulation suitable for the individual;
(f) means for generating electronic manufacturing instructions to control the operation of composition manufacturing apparatus in accordance with the customised composition; and
(g) a composition product manufacturing apparatus.

35. Use of a computer system as defined in claim 32 to make a customised composition.

36. A visual display (e.g. a screen or VDU) showing a plurality of shapes (e.g. rings), wherein:
(a) at least one of the shapes has a distinctive (and/or detectable) feature for detection (e.g. at least one of the rings is an incomplete circle, such as having a gap), and is of a colour within the wavelength range of 460-495 nm;
(b) the main or background is of a colour within the wavelength of 460-495 nm; and/or
(c) there are four shapes (e.g. rings), only one of which has a distinctive (and/or detectable) feature.
